# EUROPÄISCHE PATENTSCHRIFT

(11) **EP 2 203 133 B1**
(45) Veröffentlichungstag und Bekanntmachung des Hinweises auf die Patenterteilung: **25.04.2012**
(21) Anmeldenummer: 08802544.0
(22) Anmeldetag: 24.09.2008
(51) Int. Cl.: A61F 2/44, A61B 17/70, A61B 17/84, A61F 2/00, A61B 17/68, A61F 2/30

(54) **FACETTENGELENKIMPLANTAT**
FACET JOINT IMPLANT
IMPLANT D'ARTICULATION FACETTAIRE

(30) Priorität: 30.10.2007 DE 102007051783
(43) Veröffentlichungstag der Anmeldung: 07.07.2010
(73) Patentinhaber: Aesculap AG, 78532 Tuttlingen (DE)
(72) Erfinder: FISCHER, Kay, 78532 Tuttlingen (DE); SCHUMACHER, Jörg, 14513 Teltow (DE); BEGER, Jens, 78532 Tuttlingen (DE)
(74) Vertreter: Hoeger, Stellrecht & Partner Patentanwälte
(86) Internationale Anmeldenummer: PCT/EP2008/008060
(87) Internationale Veröffentlichungsnummer: WO 2009/056199

(56) Entgegenhaltungen:
- WO-A-2006/009855
- WO-A-2006/102443
- WO-A-2007/106573
- DE-U1-202007 015 080
- US-A1- 2005 177 240
- US-A1- 2006 190 081

## Beschreibung

Die Erfindung betrifft ein Facettengelenkimplantat mit zwei im Bereich einer Facettenanlagefläche an die Außenfläche einer Facette anlegbaren Komponenten und mit einem Verbindungsmittel, durch das die beiden Komponenten durch einen Kanal in einer Facette oder in zwei aneinander liegenden Facetten hindurchgehend miteinander verbunden sind.

Die Wirbelgelenke oder Facettengelenke tragen häufig zu einem großen Teil zum Rückenschmerz bei. Durch degenerative Veränderungen und Abnutzung der Gelenkflächen kann es zu erhöhtem Druck auf die Nervenendigungen kommen und damit zur Schmerzentstehung. Die zunehmende Instabilität des Facettengelenkes kann dabei durch entsprechende Kompensationsmechanismen zu einer Hypertrophierung des Facettengelenkes führen mit der Folge einer Spinalkanalstenose oder einer Foramenstenose. In diesem Falle werden häufig ein Teil der Lamina und des Facettengelenkes entfernt, außerdem ist es üblich, das Segment zu fusionieren, um eine Instabilität zu verhindern.

Schäden am Facettengelenk verhindern häufig auch den Einsatz von Bandscheibenprothesen, da diese nur bei intakten Facettengelenken sinnvoll eingesetzt werden können.

Es ist bekannt, Facettengelenke durch Implantate zu ersetzen, dabei werden die Implantate häufig über komplizierte mechanische Konstruktionen an den Facetten gehalten. So ist es beispielsweise bekannt, Facettengelenkimplantate in den Körper einzupflanzen, die aus zwei Komponenten bestehen, die zwischen sich eine Facette oder zwei aneinander liegende Facetten aufnehmen und die dadurch miteinander verbunden sind, dass durch einen Kanal in der Facette oder in den aneinander liegenden Facetten Stäbe oder Schrauben hindurchgreifen, an denen die beiden Komponenten festgelegt sind (US 2006/0036323 A1; US 2005/0049705 A1; US 2005/0085912 A1; US 2006/0085075 A1). Die Verbindung über Stäbe oder Schrauben macht das Einführen des Implantates in den Körper schwierig und außerdem sind diese Verbindungsmittel in Form von Schrauben oder Stäben nur schwer mit den Komponenten zu verbinden, das Gegeneinanderspannen der Komponenten benötigt viel Raum im Operationsgebiet und führt daher zu großen Zugangsöffnungen.

Dokument US2005177240A offenbart ein Implantat gemäss dem Oberbegriff von Anspruch 1. Es ist Aufgabe der Erfindung, ein gattungsgemäßes Facettengelenkimplantat so auszubilden, dass seine Implantation und sein Anlegen an die Facette oder die Facetten erleichtert wird.

Diese Aufgabe wird bei einem Facettengelenkimplantat der eingangs beschriebenen Art erfindungsgemäß dadurch gelöst, dass das Verbindungsmittel als flexibler Faden ausgebildet ist, der die beiden Komponenten derart gegeneinander spannt, dass die Komponenten gegen die Außenfläche der Facette gedrückt werden, an der sie anliegen.

Die Verwendung eines flexiblen Fadens als Verbindungsmittel ermöglicht das Einbringen der Implantatkomponenten und des Verbindungsmittels durch einen sehr kleinen Zugang, und auch das Gegeneinanderspannen der beiden Komponenten kann in einfacher Weise dadurch erfolgen, dass der die beiden Komponenten verbindende Faden durch einen Kanal in der Facette oder in den beiden Facetten hindurchgezogen und gespannt wird. Dazu ist nur ein geringer Raum im Bereich der Implantatkomponenten notwendig, da der Faden umgelenkt werden kann und die Zugkräfte dann in beliebiger Richtung aufgebracht werden können.

Der Faden kann dabei auch die Form eines Drahtes oder eines Seiles oder dergleichen haben, wesentlich ist, dass es sich um ein flexibles, fadenförmiges Zugmittel handelt. Beispielsweise könnte als Faden ein chirurgischer Nähfaden verwendet werden.

Eine der Komponenten ist so klein dimensioniert, dass sie durch den Kanal in der Facette bzw. den Facetten hindurchsteckbar ist. Auf diese Weise ist die Implantation des Implantates von einer Seite einer Facette ausgehend möglich, von dieser einen Seite ausgehend wird zunächst die hindurchsteckbare Komponente durch den Kanal in der Facette hindurchgesteckt bis auf die gegenüberliegende Seite der Facette und dort dann quer zum Kanal angeordnet, so dass diese Komponente als Rückhalteelement wirkt, das an der Außenseite der Facette anliegt. Zwischen den Facetten läuft dann der Faden durch den Kanal und kann gespannt werden, so dass die beiden Komponenten auf gegenüberliegenden Seiten des Kanals gegen die Außenflächen der Facette gezogen werden.

Die hindurchsteckbare Komponente ist ein schmales, längliches Bauteil, dessen Länge größer ist als der Durchmesser des Kanals. Beispielsweise kann die hindurchsteckbare Komponente eine im Wesentlichen rechteckige Form aufweisen.

Es ist günstig, wenn die hindurchsteckbare Komponente derart gebogen ist, dass sie eine konkave Facettenanlagefläche und eine konvexe Außenfläche aufweist. Dadurch legt sich diese Komponente mit der Facettenanlagefläche flächig an die Außenseite der Facette an und trägt auch nach außen durch die konvexe Außenfläche wenig gegenüber der Facette auf.

Bei einer besonders bevorzugten Ausführungsform ist vorgesehen, dass an einer Stirnseite der hindurchsteckbaren Komponente ein Zugglied angreift. Dabei kann es sich beispielsweise um einen oder mehrere Fäden handeln oder auch um eine Nadel, die gegebenenfalls auch unter Zwischenschaltung eines Fadens mit der durchsteckbaren Komponente verbunden ist. Dieses Zugglied kann zunächst durch den Kanal in der Facette hindurchgesteckt werden, beispielsweise mit Hilfe eines Nadelhalters, und dann kann über das Zugglied die durchsteckbare Komponente durch den Kanal in der Facette nachgezogen werden. Dies erleichtert die Einführung und das Durchschieben der hindurchsteckbaren Komponente.

Besonders vorteilhaft ist es, wenn der die beiden Komponenten verbindende Faden zwischen den beiden Komponenten mehrfach hin- und her läuft und zumindest an einer Komponente verschieblich umgelenkt wird, so dass eine Flaschenzuganordnung entsteht. Dadurch können mit relativ geringen Zugkräften an dem Faden hohe Spannkräfte erzeugt werden, mit denen die beiden Komponenten des Facettengelenkimplantates gegen die Außenflächen der jeweiligen Facette gedrückt werden.

So kann vorgesehen werden, dass mindestens eine der Komponenten als Umlenkung für den Faden mindestens zwei nebeneinander liegende Öffnungen aufweist.

Bei einer bevorzugten Ausführungsform weist mindestens eine der Komponenten eine zentrale Öffnung für den Faden oder die Fäden auf.

Es ist günstig, wenn mindestens eine Komponente einen Zentriervorsprung trägt, der bei an der Facette anliegender Komponente in den Kanal eintaucht.

Dadurch wird die Komponente gegen eine seitliche Verschiebung gesichert und in eine genau definierte Anlageposition an der Facette geführt, wenn sie gegen die Facette gespannt wird.

Der Zentriervorsprung kann bei einer bevorzugten Ausführungsform als Hülse ausgebildet sein, die eine Öffnung in der Komponente umgibt, durch die der Faden oder die Fäden hindurchgeführt sind.

Es ist günstig, wenn der Innenraum der Hülse mit mindestens einem radial von der Hülse abgehenden Fadenkanal in Verbindung steht, auf diese Weise kann der Faden in der Ebene der Facettengelenkflächen verlaufend nach au-Ben geführt werden.

Bei einer ersten bevorzugten Ausführungsform ist vorgesehen, dass die an der Innenseite einer Facette anlegbare Komponente auf ihrer der Facettenanlagefläche gegenüberliegenden Seite als Facettengelenkfläche ausgebildet ist. Da- - mit kann diese Komponente eine Gelenkfläche des Facettengelenkes ersetzen.

Ein solches Implantat kann nur an einer Facette angeordnet werden, so dass nur eine Gelenkfläche der Facette ersetzt wird, es ist aber auch möglich, derartige Implantate an beiden zu einem Facettengelenk gehörenden facetten anzuordnen, so dass dann die innen liegenden Komponenten die beiden Gelenkflächen des Facettengelenkes ersetzen und aneinander anliegen. Vorzugsweise liegen diese Komponenten dann gelenkig aneinander an, insbesondere kann vorgesehen sein, dass die Facettengelenkfläche der innen liegenden Komponente eine ballige Vertiefung oder eine ballige Erhöhung aufweist zur Anlage an einer balligen Erhöhung bzw. balligen Vertiefung einer Facettengelenkfläche eines zweiten Facettengelenkimplantates desselben Facettengelenkes.

Die Komponenten des Implantates können auch an den Außenseiten von zwei Facetten anliegen, die an ihrer Innenseite über ein natürliches Facettengelenk aneinander anliegen, in diesem Falle werden die beiden Facetten durch ein solches Implantat dauerhaft so gegeneinander gespannt, dass eine Fusion der Facetten im Gelenkbereich ermöglicht wird.

Bei einer bevorzugten Ausführungsform ist vorgesehen, dass die an der Außenseite einer Facette anlegbare Komponente von einem Rahmen umgeben ist, an dem sich die Komponente abstützt und durch den die Anlagefläche der Komponente an der Außenseite der Facette vergrößert wird. Dieser Rahmen kann sehr verschiedene Formen haben, wesentlich ist lediglich, dass sich die Komponente an ihm abstützt und dass durch den Rahmen die Anlagefläche an der Facette vergrößert wird.

Der Rahmen kann beispielsweise die Komponente vollständig umgeben.

Es ist auch möglich, dass der Rahmen eine seitliche Durchbrechung aufweist, durch die der Faden oder die Fäden hindurchtreten können, die die beiden Komponenten miteinander verbinden. Auf diese Weise ist es möglich, den Rahmen nach dem Einsetzen des Implantates einzufügen.

Es kann auch vorgesehen sein, dass der Rahmen eine die Komponente aufnehmende Vertiefung aufweist, so dass der Rahmen und die in die Vertiefung eingesetzte Komponente ein Bauteil zur Abstützung des Implantates an der Außenseite der Facette bilden, dessen Außenkontur einheitlich ist. Dadurch werden Verletzungen des umliegenden Gewebes weitgehend vermieden.

Die an der Innenseite einer Facette anlegbare Komponente kann an ihrer Facettenanlagefläche Fixiervorsprünge tragen, beispielsweise in Form von Spitzen, Spikes oder Rippen, vorzugsweise können die Rippen bei einer scheibenförmigen Komponente radial nach außen verlaufen. Diese Vorsprünge fixieren die Komponente gegen eine Verschiebung oder Verdrehung relativ zur Facette, da sie geringfügig in die Knochenoberfläche eindringen.

Bei einer bevorzugten Ausführungsform ist vorgesehen, dass der Faden insgesamt im Bereich zwischen den beiden Komponenten eine Schlaufe ausbildet und dass die Enden des Fadens mittels eines auf einem Ende des Fadens verschieblichen Spannknotens verknotet sind. Dadurch lässt sich der Faden in an sich bekannter Weise dadurch spannen, dass dieser Knoten auf dem Ende des Fadens verschoben wird, beispielsweise mittels eines Knotenschiebers, wie er auch bei der Herstellung einer Wundnaht unter Verwendung eines chirurgischen Nähfadens zum Einsatz kommt.

Die nachfolgende Beschreibung bevorzugter Ausführungsformen dient im Zusammenhang mit der Zeichnung der näheren Erläuterung.

Es zeigen:
- Figur 1:: eine perspektivische Darstellung von zwei Wirbelknochen mit einem Facettengelenkimplantat zum Ersatz einer Facettengelenkfläche;
- Figur 2:: eine vergrößerte Detailansicht des Bereiches A in figur 1;
- Figur 3:: eine perspektivische Ansicht des Facettengelenkimplantates der Figur 2 mit einer Gelenkflächenkomponente, einer Gegenhalter- komponente und einem die beiden verbindenden, flaschenzugartig geführten Faden;
- Figur 4:: eine perspektivische Ansicht des implantates der Figur 3 von der Gelenkflächenseite her gesehen;
- Figur 5:: eine perspektivische Ansicht der Facettenanlagefläche einer Gelenkflächenkomponente mit Vorsprüngen auf der Facettenanlagefläche;
- Figur 6:: eine Ansicht ähnlich Figur 2 bei Implantaten zum Ersatz von beiden Gelenkflächen eines Facettengelenkes;
- Figur 7:: eine perspektivische Ansicht der beiden Implantate der Figur 6 vor deren gegenseitiger Anlage;
- Figur 8:: eine Ansicht ähnlich Figur 3 bei einem abgewandelten Ausführungsbeispiel mit einem nadelförmigen Zugglied an der Gegenhalterkomponente;
- Figur 9:: eine Ansicht eines Facettengelenkimplantates gemäß Figur 3 mit einem die Gegenhalterkomponente umgebenden Rahmen;
- Figur 10:: eine Ansicht ähnlich Figur 9 mit einem gegenüber der Gegenhalterkomponente verschobenen Rahmen und
- Figur 11:: eine Ansicht ähnlich Figur 2 mit einem zwei Facetten gegeneinander spannenden Facettengelenkimplantat.

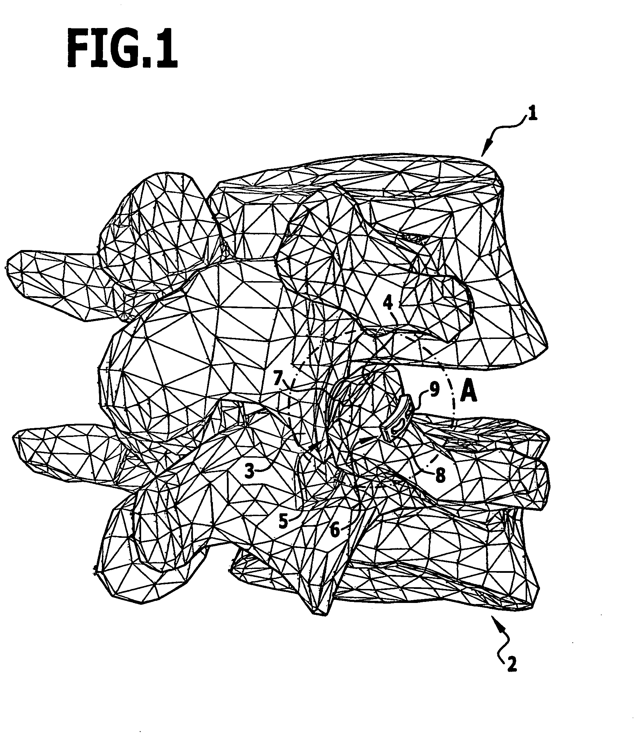

In Figur 1 sind zwei nebeneinander angeordnete Wirbelkörper 1, 2 dargestellt. Beide Wirbelkörper tragen jeweils zwei obere und zwei untere knöcherne Vorsprünge, die als Facetten 3, 4 bezeichnet werden. Jeweils eine untere Facette 3 eines oberen Wirbelkörpers 1 und eine obere Facette 4 eines unteren Wirbelkörpers 2 liegen aneinander an und bilden im Anlagebereich ein Facettengelenk 5 aus. Im Bereich des Facettengelenkes tragen beide Facetten 3, 4 Gelenkflächen, die fast eben sind und nur eine geringfügige Wölbung aufweisen und die jeweils normalerweise mit einer Knorpelschicht bedeckt sind, so dass im Bereich des Facettengelenkes 5 die Knorpelschichten der Gelenkflächen flächig aneinander anliegen.

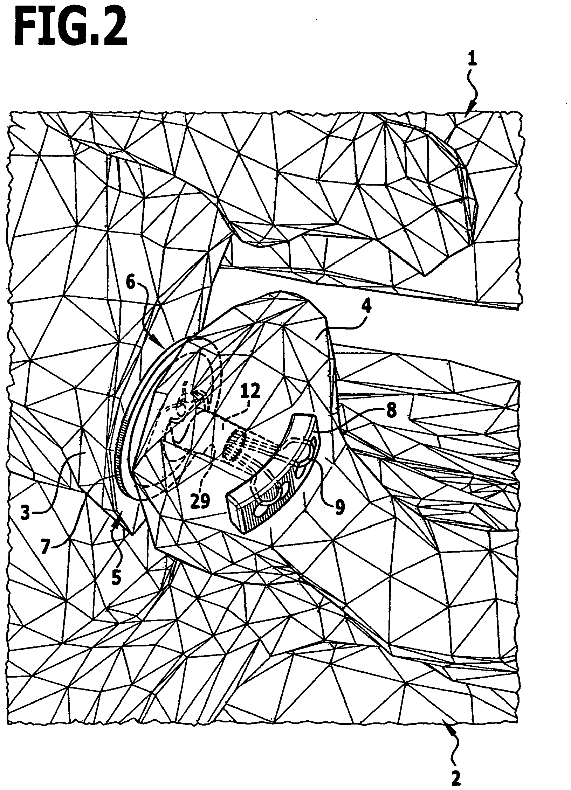

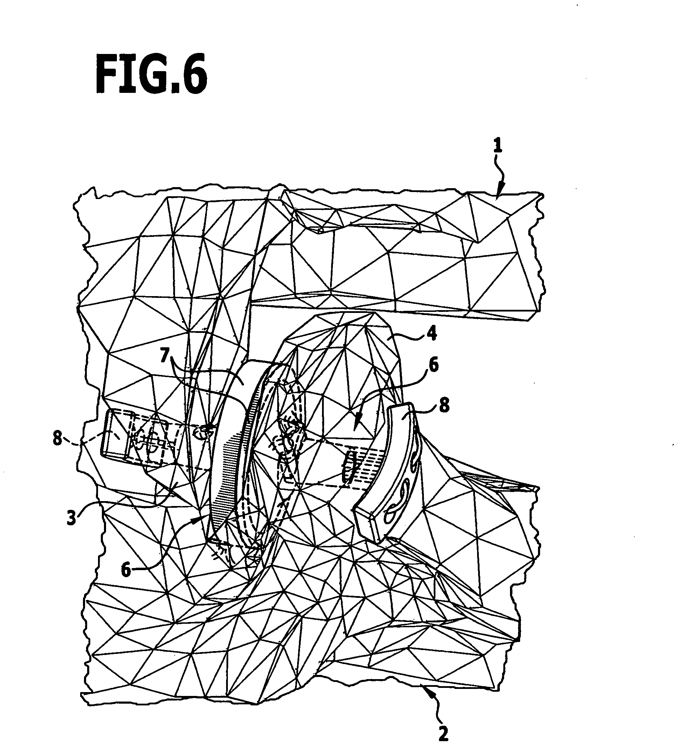

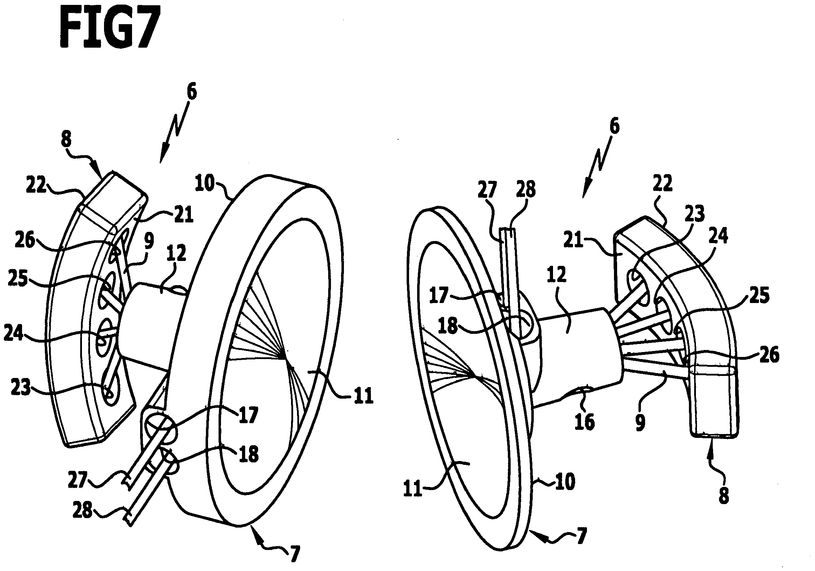

Bei dem in den Figuren 1 und 2 dargestellten Ausführungsbeispiel ist die Gelenkfläche einer oberen Facette 4 eines unteren Wirbelkörpers 2 ersetzt durch ein Facettengelenkimplantat 6, welches zwei Komponenten 7, 8 umfasst, die mittels eines Fadens 9 miteinander verbunden sind. Die erste Komponente 7 ist an der Innenseite der Facette 4 angeordnet und ersetzt die natürliche Facettengelenkfläche an dieser Innenseite der Facette 4. Dazu hat die Komponente 7 die Form einer Scheibe mit kreisförmigem Querschnitt, die mit einer der Facette 4 zugewandten Facettenanlagefläche im Bereich der natürlichen Facettengelenkfläche diese überdeckend an der Facette 4 anliegt und deren der Facettenanlagefläche 10 gegenüberliegende Außenfläche als Facettengelenkfläche 11 ausgebildet ist. Diese Facettengelenkfläche 11 kann beispielsweise eine geringfügig ballige Vertiefung aufweisen, so dass sich die natürliche Gelenkfläche der gegenüberliegenden Facette 3 oder eine ähnliche Facettengelenkfläche 11 eines gleich aufgebauten Facettengelenkimplantates an der anderen Facette 3, bei dem die Gelenkfläche in komplementärer Weise ballig erhaben ausgebildet ist, flächig an die Gelenkfläche anlegt und auf diese Weise ein teilweise oder vollständig ersetztes Facettengelenk gebildet wird. Bei dem Ausführungsbeispiel der Figuren 1 bis 4 ist jeweils nur eine Facettengelenkfläche durch ein derartiges Facettengelenkimplantat ersetzt, beim Ausführungsbeispiel der Figuren 6 und 7 dagegen trägt jede der beiden an einem Facettengelenk aneinander liegenden Facetten ein solches Facettengelenkimplantat, diese sind dabei bis auf die Wölbung der Facettengelenkflächen 11 gleich ausgebildet, die beiden Gelenkflächen sind dabei komplementär zueinander gewölbt, so dass sie flächig ineinander passen, wie dies aus der Darstellung der Figur 7 deutlich wird.

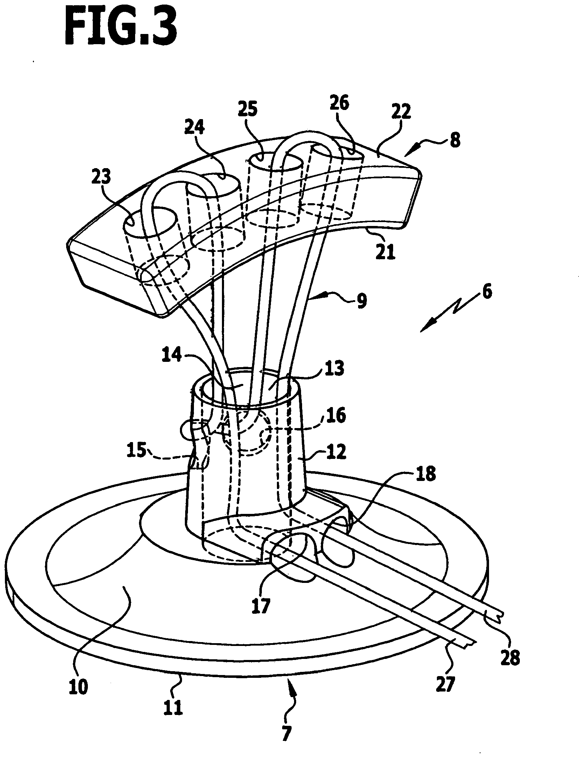

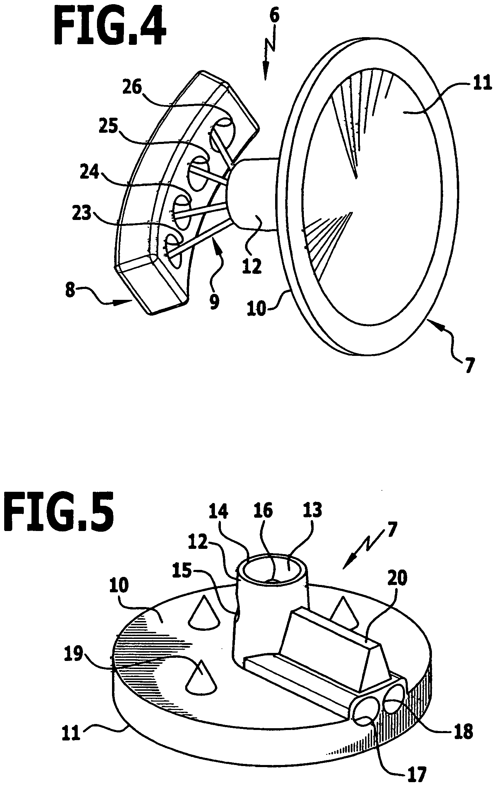

Auf der Facettenanlagefläche 10 trägt die Komponente 7 eine zentrale, mit ihrer Längsachse senkrecht auf der scheibenförmigen Komponente 7 stehende Hülse 12, deren Innenraum 13 durch die scheibenförmige Komponente 7 einseitig verschlossen ist, während die gegenüberliegende Seite offen ist. In der Seitenwand 14 der Hülse 12 sind zwei nebeneinander liegende Durchbrechungen 15 und 16 angeordnet, außerdem treten am verschlossenen Ende der Hülse 12 zwei nebeneinander liegende, radial verlaufende Fadenkanäle 17, 18 in diese ein, die bei dem Ausführungsbeispiel der Figur 3 relativ kurz ausgebildet sind, so dass sie im Abstand innerhalb des Randes der scheibenförmigen Komponente 7 enden, beim Ausführungsbeispiel der Figur 5 dagegen enden diese Fadenkanäle 17, 18 an der Außenkante der scheibenförmigen Komponente 7.

Auf der Facettenanlagefläche 10 können außerdem Fixiervorsprünge vorgesehen sein, beispielsweise in Form von Spitzen oder Spikes 19 oder in Form einer Rippe 20, die bei dem Ausführungsbeispiel der Figur 5 oberhalb der beiden Fadenkanäle 17, 18 parallel zu diesen zwischen der Hülse 12 und dem Außenrand der scheibenförmigen Komponente 7 verläuft.

Derartige Fixiervorsprünge können auch an den anderen Komponenten des Facettengelenkes vorgesehen sein und an den anderen Ausführungsbeispielen dieser Komponenten.

Die zweite Komponente 8 des Facettengelenkimplantats 6 hat die form eines langgestreckten Rechteckes oder Quaders, der in Längsrichtung gebogen ist, so dass eine konkave, innen liegende Facettenanlagefläche 21 und eine konvexe Außenfläche 22 entstehen. Die Krümmung der beiden Komponente 8 ist so gewählt, dass die Facettenanlagefläche 21 bei außenseitiger Anlage an einer Facette flächig an dieser anliegt, d.h. sie entspricht der Krümmung einer Facette im Anlagebereich.

Bei den in der Zeichnung dargestellten Ausführungsbeispielen trägt die zweite, quaderförmige Komponente 8 nebeneinander vier durchgehende Öffnungen 23, 24, 25, 26, durch die der Faden 9 hindurchgeführt ist. Bei dem dargestellten Ausführungsbeispiel tritt dieser Faden 9 zur Verbindung der beiden Komponenten 7, 8 durch einen Fadenkanal 17 in den Innenraum 13 der Hülse 12 ein und wird dann in entgegengesetzter Richtung durch zwei nebeneinander liegende Öffnungen 23, 24 der zweiten Komponente 8 hindurchgeführt. Dabei wird der Faden 9 an dieser zweiten Komponente 8 umgelenkt und liegt zwischen den beiden Öffnungen 23, 24 an der Außenfläche 22 der zweiten Komponente 8 an.

Von der zweiten Öffnung 24 tritt der Faden 9 durch den Innenraum 13 der Hülse 12 von innen nach außen durch die Durchbrechung 15 hindurch und anschließend durch die daneben liegende Durchbrechung 16 wieder in den Innenraum 13 der Hülse 12 ein, im weiteren Verlauf durchsetzt der Faden wieder gegenläufig die beiden Öffnungen 25 und 26 der zweiten Komponente 8 und gelangt von der Öffnung 26 schließlich durch den Innenraum 13 der Hülse 12 und den zweiten Fadenkanal 18 wieder nach außen. Insgesamt bildet der Faden 9 somit zwei nebeneinander liegende Schlaufen aus. Wenn man an den Enden 27, 28 des Fadens 9 zieht, führt dies dazu, dass sich der Faden 9 spannt und die beiden Komponenten 7, 8 nach Art eines Flaschenzuges gegeneinander zieht.

Zum Einsetzen des beschriebenen Facettengelenkimplantates 6 wird zunächst durch die entsprechende Facette 4 ein im Wesentlichen senkrecht auf der natürlichen Facettengelenkfläche stehender Kanal 29 gebohrt. Die Facettengelenkskapsel wird eröffnet und die Facettengelenkoberfläche wird, beispielsweise mit einer Raspel, präpariert. Es ist auch möglich, für eine beschleunigte Fusion die Knorpelschicht der Facettengelenke vollständig zu entfernen. Weiterhin kann der Bohrkanal mit Wachstumsfaktoren, z.B. BMP (bone morphogenic proteins = knochenbildende Proteine) befüllt werden. Durch den Kanal 29 wird von der Innenseite der Facette, also von der Facettengelenkseite her, die zweite Komponente 8 eines Facettengelenkimplantates 6 durch den Kanal 29 hindurchgesteckt, bis er an der Außenseite der Facette aus diesem Kanal 29 austritt. Der Faden 9 ist dabei locker, so dass die zweite Komponente 8 durch den Kanal 29 hindurchgesteckt werden kann, während die erste Komponente 7 noch außerhalb des Facettengelenkraumes verbleibt.

Anschließend wird nach Spreizung des Facettengelenkraumes die erste Komponente 7 in den Zwischenraum zwischen den zwei Facetten 3, 4 eingeführt, so dass die Hülse 12 in den Kanal 29 eintritt. Die Hülse 12 wirkt damit als Zentriervorsprung, der die Lage der ersten Komponente 7 durch Eintauchen in den Kanal 29 definiert und fixiert. Die zweite Komponente 8 wird nach dem Durchstecken durch den Kanal 29 gekippt und so angeordnet, dass sie den Austritt des Kanals 29 überdeckt und dass sie sich mit ihrer Facettenanlagefläche 21 an die Außenseite der Facette 4 anlegt.

Um die beiden Komponenten 7, 8 an der Facette festzulegen, wird nunmehr der Faden 9 gespannt. Dies kann entweder dadurch erfolgen, dass der Faden an beiden Enden 27, 28 gegenüber der zweiten Komponente 8 herausgezogen wird, es ist auch möglich, dass ein Ende 27 mit dem anderen Ende 28 so verknotet ist, dass der entstehende Knoten auf einem Ende frei verschieblich ist. Durch Verschieben des Knotens in Richtung auf die zweite Komponente 8 und durch gleichzeitiges Ziehen am anderen Ende kann der Faden 9 im Bereich zwischen den beiden Komponenten 7, 8 gespannt werden, und diese Spannung wird durch den Knoten dann aufrechterhalten. Bei dem Spannen des Fadens werden die beiden Komponenten 7, 8 jeweils an die Facette 4 angedrückt, die Spitzen 19 und die Rippe 20 drücken dabei in die Facette 4 hinein und fixieren die scheibenförmige Komponente 7 gegen jegliche Verschiebung relativ zur Facette 4. Allein durch das Spannen des Fadens werden also die beiden Komponenten 7, 8 fest und dauerhaft an der Facette 4 festgelegt.

Wird das Facettengelenk 5 nur an einer Gelenkfläche ersetzt, wird nur ein derartiges Implantat an einer Facette festgelegt, sollen beide Gelenkflächen ersetzt werden, wird jeweils ein entsprechendes Implantat an jeder Facette festgelegt, wobei sich dann die Facettengelenkflächen der beiden Implantate gelenkig und flächig aneinander anlegen, wie dies anhand der Figur 7 erläutert worden ist.

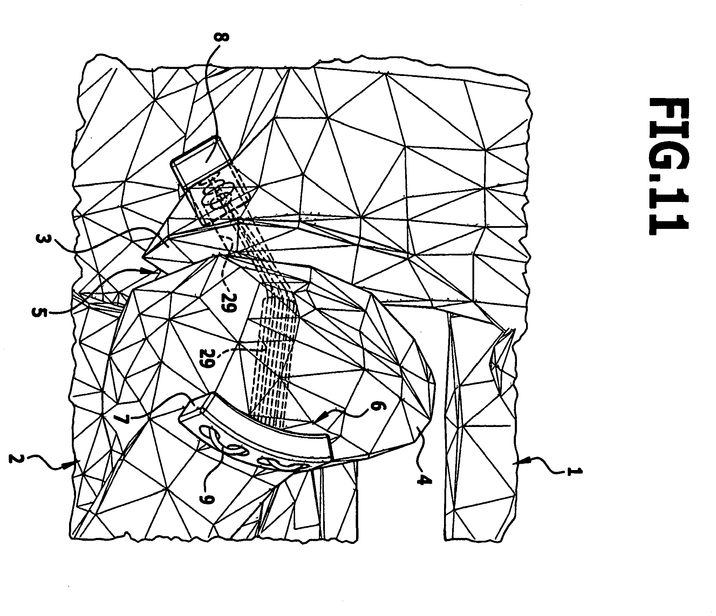

Wenn die beiden Facetten 3, 4 dauerhaft fest miteinander verbunden werden sollen, wenn also eine Fusion der beiden Facetten gewünscht wird, dann werden beide Facetten mit einem entsprechenden Kanal 29 durchbohrt, wobei die Kanäle zueinander ausgerichtet sind, und dann wird ein ähnliches Facettengelenkimplantat 6 eingesetzt, welches jedoch im Unterschied zu dem bisher beschriebenen Facettengelenkimplantat zwei Komponenten aufweist, die beide gleich ausgebildet sind wie die zweite Komponente 8 des Facettengelenkimplantates der Figuren 1 bis 4. Es fehlt also bei diesem Implantat dann eine Komponente, die scheibenförmig ausgebildet ist und eine Gelenkfläche ersetzt, beide Komponenten sind wie die außen liegende Komponente 8 des Ausführungsbeispiels der Figuren 1 bis 4 als reine Halteelemente ausgebildet, die an der Außenseite der Facetten angreifen und die beim Spannen des Fadens dann die beiden Facetten dauerhaft gegeneinander drücken, wie dies in Figur 11 dargestellt ist.

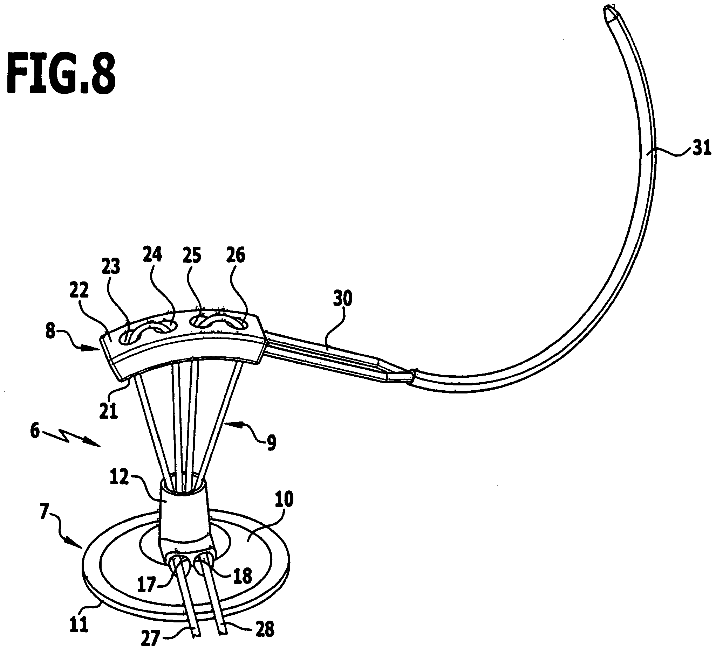

Das Einführen der länglichen, rechteckigen, außen liegenden Komponente 8 kann dadurch erleichtert werden, dass an dieser an einer Stirnfläche ein Faden 30 und eine vorzugsweise gebogene Nadel 31 angeordnet sind, wie dies in Figur 8 dargestellt ist. Beim Einsetzen wird zunächst, beispielsweise mit Hilfe eines Nadelhalters, die Nadel 31 von der Facettengelenkseite her durch den Kanal 29 hindurchgezogen, der mit der Nadel 31 verbundene Faden 30 zieht dann die längliche Komponente 8 hinter sich her durch den Kanal 29 hindurch, so dass der Operateur keine Schwierigkeit hat, die Komponente 8 auf der Gelenkseite in den Kanal 29 einzuführen.

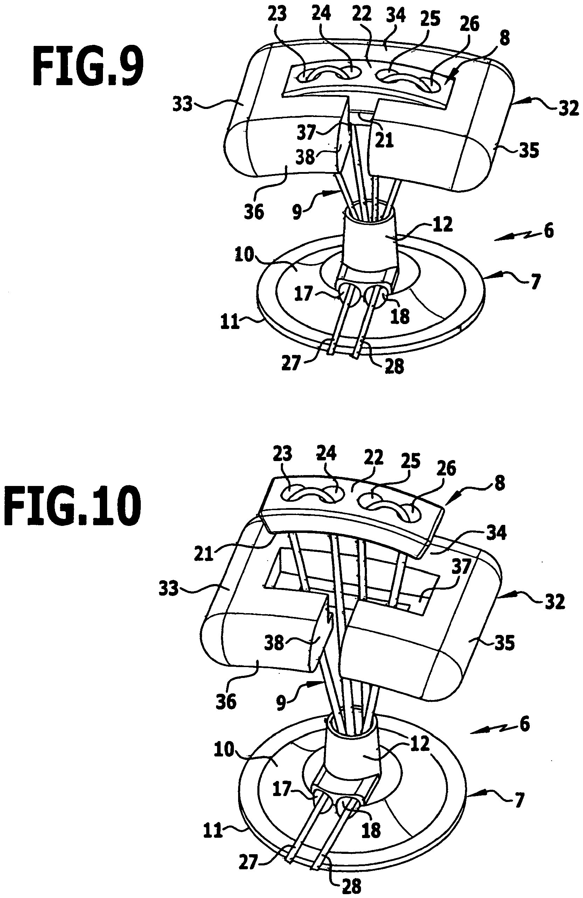

Die Querschnittsfläche der außen liegenden zweiten Komponente 8 ist normalerweise relativ klein gewählt, damit diese außen liegende Komponente 8 durch den Kanal 29 der Facette hindurchgesteckt werden kann. Dadurch ergeben sich unter Umständen relativ kleine Andruckflächen an der Außenseite der Facette. Um diese Anlageflächen zu vergrößern, kann ein Rahmen 32 vorgesehen sein, der die Komponente 8 umgibt und der dadurch ihre Anlagefläche an der Facette vergrößert. Dieser Rahmen 32 kann beispielsweise, wie dies in Figur 9 dargestellt ist, außen liegende Schenkel 33, 34, 35, 36 aufweisen, die eine Vertiefung 37 umgeben, die ihrerseits einen Aufnahmeraum für die außen liegende zweite Komponente 8 bildet. Seitlich kann diese Vertiefung 37 im Bereich eines Schenkels 36 einen Spalt 38 ausbilden, durch den der Faden 9 hindurchgeführt werden kann, wenn die Komponente 8 von oben her in die Vertiefung 37 eingesetzt wird. Bei einem solchen Rahmen kann nach dem Einsetzen des Facettengelenkimplantates in der oben beschriebenen Weise und vor dem Spannen des Fadens dieser Rahmen an der Außenseite der Facette seitlich so an den Faden 9 und die außen liegende Komponente 8 herangeschoben werden, dass ein Einsetzen der Komponente 8 in die Vertiefung 37 möglich wird und dass diese nach dem Einsetzen diese Vertiefung vollständig ausfüllt, wie dies in Figur 9 dargestellt ist. Beim Spannen des Fadens wird dann der Rahmen 32 mit der darin aufgenommenen außen liegenden Komponente 8 gegen die Außenseite der Facette 4 gezogen, und dadurch wird die Anlagefläche an der Außenseite der Facette vergrößert gegenüber der relativ kleinen Anlagefläche der außen liegenden Komponente 8 allein.

Der Rahmen 32 kann natürlich auch andere Formen aufweisen, beispielsweise die Form einer etwas größeren Platte.

Es gibt Fälle, in denen Teile der Facette reseziert werden müssen, und dann kann der Rahmen 32 auch als Defektauffüllung dienen. In diesem Falle wird der Rahmen vorzugsweise entsprechend der Form des Defektes ausgebildet und in diesen eingesetzt, so dass das fehlende Knochenmaterial der Facette durch den Rahmen ersetzt wird.

Der Rahmen kann in diesem Falle beispielsweise das Facettengelenk auf Biegebelastungen stabilisieren.

Es ist dabei vorteilhaft, wenn der Rahmen aus einem knochenverträglichen Material besteht, beispielsweise einem Metall, einem beschichteten Metall oder einem Polymer oder aber auch eine poröse Metallstruktur aufweist, in die der Knochen einwachsen kann. Auf diese Weise übernimmt der Rahmen eine Doppelfunktion einmal als Defektauffüllung und zum anderen als Gegenlager für den Faden 9 zum Gegeneinanderspannen der beiden Komponenten 7, 8 des Facettengelenkes 6.

Die Komponenten können aus Metall, Keramik oder Polymeren hergestellten werden, beispielsweise aus Polyetheretherketon (PEEK), aus Polyethylen (PE) oder aus kohlefaserverstärktem Polyetheretherketon (CFR PEEK). Die Polymere können eine osteokonduktive Beschichtung aus Metall oder Keramik besitzen, so dass die Verbindung mit dem angrenzenden Knochenmaterial gefördert wird.

Denkbare Materialkombinationen für die Facettengleitflächen sind:
- Metall / Polymer: z.B. CoCroMo / ultrahochmolekulargewichtiges Polyethylen (UHMWPE)
   CoCroMo / kohlefaserverstärktes Polyethylen (CFR PEEK)
- Polymer / Polymer: z.B. CFR PEEK / CFR PEEK
- Keramik / Keramik: z.B. Titan keramikbeschichtet / Titan keramikbeschichtet
- Hydrogelbeschichtungen auf Metall oder Polymer.

## Patentansprüche

1. Facettengelenkimplantat (6) mit zwei im Bereich einer Facettenanlagefläche (10) an die Außenfläche einer Facette (3, 4) anlegbaren Komponenten (7, 8) und mit einem Verbindungsmittel (9), durch das die beiden Komponenten (7, 8) durch einen Kanal in einer Facetten oder in zwei aneinander liegenden Facetten (3, 4) hindurchgehend miteinander verbunden sind, wobei das Verbindungsmittel als flexibler Faden (9) ausgebildet ist, der die beiden Komponenten (7, 8) derart gegeneinander spannt, dass die Komponenten (7, 8) gegen die Außenfläche der Facette (3, 4) gedrückt werden, an der sie anliegen, und wobei mindestens eine der Komponenten (8) so klein dimensioniert ist, dass sie durch den Kanal (29) in der Facette beziehungsweise den Facetten (3, 4) hindurchsteckbar ist, **dadurch gekennzeichnet, dass** die hindurchsteckbare Komponente (8) ein schmales, längliches Bauteil ist, dessen Länge größer ist als der Durchmesser des Kanals (29).

2. Facettengelenkimplantat nach Anspruch 1, **dadurch gekennzeichnet, dass** an einer Stirnseite der hindurchsteckbaren Komponente (8) ein Zugglied (30, 31) angreift.

3. Facettengelenkimplantat nach Anspruch 2, **dadurch gekennzeichnet, dass** das Zugglied einen oder mehrere Fäden (30) aufweist.

4. Facettengelenkimplantat nach Anspruch 2 oder 3, **dadurch gekennzeichnet, dass** das Zugglied eine Nadel (31) aufweist.

5. Facettengelenkimplantat nach einem der voranstehenden Ansprüche, **dadurch gekennzeichnet, dass** der die beiden Komponenten (7, 8) verbindende Faden (9) zwischen den beiden Komponenten (7, 8) mehrfach hin- und her läuft und zumindest an einer Komponente (8) verschieblich umgelenkt wird, so dass eine Flaschenzuganordnung entsteht.

6. Facettengelenkimplantat nach Anspruch 5, **dadurch gekennzeichnet, dass** mindestens eine der Komponenten (8) als Umlenkung für den Faden (9) mindestens zwei nebeneinander liegende Öffnungen (23, 24, 25, 26) aufweist.

7. Facettengelenkimplantat nach einem der voranstehenden Ansprüche, **dadurch gekennzeichnet, dass** mindestens eine der Komponenten (7) eine zentrale Öffnung (13) für den Faden (9) oder die Fäden aufweist.

8. Facettengelenkimplantat nach einem der voranstehenden Ansprüche, **dadurch gekennzeichnet, dass** mindestens eine Komponente (7) einen Zentriervorsprung (12) trägt, der bei an der Facette (3, 4) anliegender Komponente (7) in den Kanal (29) eintaucht.

9. Facettengelenkimplantat nach Anspruch 8, **dadurch gekennzeichnet, dass** der Zentriervorsprung als Hülse (12) ausgebildet ist, die eine Öffnung (13) in der Komponente (7) umgibt, durch die der Faden (9) oder die Fäden hindurchgeführt sind.

10. Facettengelenkimplantat nach Anspruch 9, **dadurch gekennzeichnet, dass** der Innenraum (13) der Hülse (12) mit mindestens einem radial von der Hülse (12) abgehenden Fadenkanal (17, 18) in Verbindung steht.

11. Facettengelenkimplantat nach einem der voranstehenden Ansprüche, **dadurch gekennzeichnet, dass** die an der Innenseite der Facette (3, 4) anlegbare Komponente (7) auf ihrer der Facettenanlagefläche (10) gegenüberliegenden Seite als Facettengelenkfläche (11) ausgebildet ist.

12. Facettengelenkimplantat nach Anspruch 11, **dadurch gekennzeichnet, dass** die Facettengelenkfläche (11) eine ballige Vertiefung oder eine ballige Erhöhung aufweist zur Anlage an einer balligen Erhöhung bzw. balligen Vertiefung einer Facettengelenkfläche (11) eines zweiten Facettengelenkimplantates (6).

13. Facettengelenkimplantat nach einem der voranstehenden Ansprüche, **dadurch gekennzeichnet, dass** die an der Außenseite einer Facette (3, 4) anlegbare Komponente (8) von einem Rahmen (32) umgeben ist, an dem sich die Komponente (8) abstützt und durch den die Anlagefläche der Komponente (8) an der Außenseite der Facette (3, 4) vergrößert wird.

## Claims

1. Facet joint implant (6) comprising two components (7, 8) adapted in the area of a facet-contacting surface (10) for placement on the outer surface of a facet (3, 4), and a fastener (9) for joining the two components (7, 8) to each other through a channel in a facet or in two facets (3, 4) lying against each other, the fastener being configured as a flexible thread (9) which tensions the two components (7, 8) towards each other such that the components (7, 8) are pressed against the outer surface of the facet (3, 4) against which they lie, and at least one of the components (8) being of such small dimensions that it is insertable through the channel (29) in the facet or facets (3, 4), **characterised in that** the insertable component (8) is a narrow, elongate component whose length is greater than the diameter of the channel (29).

2. Facet joint implant in accordance with claim 1, **characterised in that** a pull member (30, 31) engages an end face of the insertable component (8).

3. Facet joint implant in accordance with claim 2, **characterised in that** the pull member comprises one or more threads (30).

4. Facet joint implant in accordance with claim 2 or 3, **characterised in that** the pull member comprises a needle (31).

5. Facet joint implant in accordance with any one of the preceding claims, **characterised in that** the thread (9) joining the two components (7, 8) runs back and forth several times between the two components (7, 8) and is displaceably deflected at least on one component (8) so as to produce a pulley assembly.

6. Facet joint implant in accordance with claim 5, **characterised in that** at least one of the components (8) has at least two adjacent openings (23, 24, 25, 26) as deflection for the thread (9).

7. Facet joint implant in accordance with any one of the preceding claims, **characterised in that** at least one of the components (7) has a central opening (13) for the thread (9) or threads.

8. Facet joint implant in accordance with any one of the preceding claims, **characterised in that** at least one component (7) carries a centering projection (12) which enters the channel (29) when the component (7) lies against the facet (3, 4).

9. Facet joint implant in accordance with claim 8, **characterised in that** the centering projection is constructed as a sleeve (12) surrounding an opening (13) in the component (7), through which the thread (9) or threads are led.

10. Facet joint implant in accordance with claim 9, **characterised in that** the interior (13) of the sleeve (12) is in communication with at least one thread channel (17, 18) radiating radially from the sleeve (12).

11. Facet joint implant in accordance with any one of the preceding claims, **characterised in that** the component (7) adapted for placement on the inner side of the facet (3, 4) is constructed as facet joint surface (11) on its side opposed to the facet-contacting surface (10).

12. Facet joint implant in accordance with claim 11, **characterised in that** the facet joint surface (11) has a spherical depression or a spherical elevation for placement on a spherical elevation or a spherical depression, respectively, of a facet joint surface (11) of a second facet joint implant (6).

13. Facet joint implant in accordance with any one of the preceding claims, **characterised in that** the component (8) adapted for placement on the outer side of a facet (3, 4) is surrounded by a frame (32) on which the component (8) is supported and by which the contacting surface of the component (8) on the outer side of the facet (3, 4) is enlarged.

## Revendications

1. Implant d'articulation facettaire (6), comprenant deux composants (7, 8) qui, dans la zone d'une surface d'appui de facette (10), peuvent être appliqués contre la surface extérieure d'une facette (3, 4), et comprenant un moyen de liaison (9) par lequel les deux composants (7, 8) sont reliés l'un à l'autre en passant à travers un canal dans une facette ou deux facettes (3, 4) appliquées l'une contre l'autre, le moyen de liaison étant réalisé en tant que fil flexible (9) qui serre les deux composants (7, 8) l'un contre l'autre de manière telle que les composants (7, 8) soient pressés contre la surface extérieure de la facette (3, 4) contre laquelle ils s'appliquent, et au moins l'un des composants (8) étant dimensionné de façon à être suffisamment petit pour pouvoir être enfilé à travers le canal (29) dans la facette ou respectivement les facettes (3, 4), **caractérisé en ce que** le composant (8) pouvant être enfilé à travers le canal est une pièce étroite, allongée, dont la longueur est plus grande que le diamètre du canal (29).

2. Implant d'articulation facettaire selon la revendication 1, **caractérisé en ce que** sur un côté frontal du composant (8) pouvant être enfilé, agit un organe de traction (30, 31).

3. Implant d'articulation facettaire selon la revendication 2, **caractérisé en ce que** l'organe de traction comprend un ou plusieurs fils (30).

4. Implant d'articulation facettaire selon la revendication 2 ou la revendication 3, **caractérisé en ce que** l'organe de traction comprend une aiguille (31).

5. Implant d'articulation facettaire selon l'une des revendications précédentes, **caractérisé en ce que** le fil (9), qui relie les deux composants (7, 8), s'étend plusieurs fois en aller-retour entre les deux composants (7, 8) et subit un renvoi coulissant sur au moins un composant (8), de sorte qu'il en résulte un agencement du type mouflage.

6. Implant d'articulation facettaire selon la revendication 5, **caractérisé en ce que** l'un au moins des composants (8) présente, en guise de renvoi pour le fil (9), au moins deux ouvertures (23, 24, 25, 26) situées côte à côte.

7. Implant d'articulation facettaire selon l'une des revendications précédentes, **caractérisé en ce que** l'un au moins des composants (7) présente une ouverture centrale (13) pour le fil (9) ou les fils.

8. Implant d'articulation facettaire selon l'une des revendications précédentes, **caractérisé en ce que** l'un au moins des composants (7) porte une protubérance de centrage (12) qui, lorsque le composant (7) est appliqué contre la facette (3, 4), s'engage dans le canal (29).

9. Implant d'articulation facettaire selon la revendication 8, **caractérisé en ce que** la protubérance de centrage est réalisée sous la forme d'une douille (12), qui entoure une ouverture (13) dans le composant (7), à travers laquelle sont menés et guidés le fil (9) ou les fils.

10. Implant d'articulation facettaire selon la revendication 9, **caractérisé en ce que** l'espace intérieur (13) de la douille (12) est en liaison avec au moins un canal de fil (17, 18) partant radialement de la douille (12).

11. Implant d'articulation facettaire selon l'une des revendications précédentes, **caractérisé en ce que** le composant (7) pouvant être appliqué contre le côté intérieur de la facette (3, 4), est réalisé, sur son côté opposé à la surface d'appui de facette (10), sous forme de surface d'articulation facettaire (11).

12. Implant d'articulation facettaire selon la revendication 11, **caractérisé en ce que** la surface d'articulation facettaire (11) présente un creux de forme bombée ou une proéminence de forme bombée, pour venir en appui sur une proéminence de forme bombée respectivement un creux de forme bombée d'une surface d'articulation facettaire (11) d'un deuxième implant d'articulation facettaire (6).

13. Implant d'articulation facettaire selon l'une des revendications précédentes, **caractérisé en ce que** le composant (8) pouvant venir s'appliquer contre le côté extérieur d'une facette (3, 4) est entouré par un cadre (32) sur lequel s'appuie le composant (8) et qui augmente la surface d'appui du composant (8) sur le côté extérieur de la facette (3, 4).
